# EUROPÄISCHE PATENTSCHRIFT

(11) **EP 2 691 306 B1**
(45) Veröffentlichungstag und Bekanntmachung des Hinweises auf die Patenterteilung: **29.03.2017**
(21) Anmeldenummer: 12715316.1
(22) Anmeldetag: 17.02.2012
(51) Int. Cl.: B65B 55/02, B67C 7/00, A61L 2/14

(54) **VERFAHREN UND VORRICHTUNG ZUM STERILISIEREN UND ZUR BLASFORMUNG VON VORFORMLINGEN**
METHOD AND APPARATUS FOR THE BLOW MOULDING AND STERILIZING OF PREFORMS
PROCÉDÉ ET ET DISPOSITIF DE STÉRILISATION ET DE MOULAGE PAR SOUFFLAGE DE PRÉFORMES

(30) Priorität: 29.03.2011 DE 102011016448
(43) Veröffentlichungstag der Anmeldung: 05.02.2014
(73) Patentinhaber: KHS Corpoplast GmbH, 22145 Hamburg (DE)
(72) Erfinder: HEROLD, Thomas, 22926 Ahrensburg (DE); RIEGER, Harald, 22303 Hamburg (DE)
(74) Vertreter: Hausfeld, Norbert
(86) Internationale Anmeldenummer: PCT/DE2012/000192
(87) Internationale Veröffentlichungsnummer: WO 2012/130197

(56) Entgegenhaltungen:
- WO-A1-99/17334
- WO-A1-2011/138463
- WO-A2-2004/024577
- WO-A2-2008/015358
- DE-A1-102007 041 573

## Beschreibung

Die Erfindung betrifft ein Verfahren zum Sterilisieren von Vorformlingen aus einem thermoplastischen Material, die zur Herstellung von blasgeformten Behältern vorgesehen sind.

Die Erfindung betrifft darüber hinaus eine Vorrichtung zur Blasformung von mindestens bereichsweise sterilen Behältern, die mindestens eine auf einer Tragstruktur angeordnete Blasstation zur Umformung von thermoplastischen Vorformlingen in die Behälter aufweist.

Eine Herstellung von sterilen blasgeformten Behältern erfolgt typischerweise derart, daß diese Behälter nach ihrer Blasformung und vor einer Befüllung unter Verwendung von Wasserstoffperoxid oder anderen Chemikalien sterilisiert werden. Ebenfalls ist es bereits bekannt, die bei der Blasformung der Behälter als Ausgangsprodukt verwendeten Vorformlinge zu sterilisieren, insbesondere den Bereich der inneren Oberfläche dieser Vorformlinge.

Es ist ebenfalls bereits bekannt, daß bei der Durchführung einer Beschichtung von Behältern unter Anwendung eines Plasmas Sterilisierungseffekte auftreten. Aufgrund der üblicherweise vorgesehenen Konturen im Bereich der Behälterwandungen treten bei derartigen Sterilisierungen aber sogenannten Abschattungen auf, die einer zuverlässigen Sterilisierung entgegenstehen.

In der WO 2009/026869 wird eine Sterilisiereinrichtung für Vorformlinge beschrieben, bei der die Sterilisierung unter Verwendung eines Plasmas durchgeführt wird. Die Sterilisiereinrichtung ist entlang eines Transportweges der Vorformlinge angeordnet, der sich von einer Zuführeinrichtung für die Vorformlinge bis zu einem Blasrad erstreckt.

Aus DE 10 2007 041 573 A1 ist ein Verfahren und eine Vorrichtung zum Sterilisieren von Vorformlingen bekannt, bei denen der Vorformling gemeinsam mit einer Sterilisierungseinrichtung entlang eines Transportweges bewegt wird und die Sterilisierung als Teil des Transportvorganges der Vorformlinge durchgeführt wird.

Aus WO 2004/024577 A2 ist ein Behälter aus Kunststoff zur Verpackung von Produkten bekannt, bei dem mit Hilfe eines in den Behälter eingeführten Plasmas eine Barriereschicht auf den inneren Oberflächen abgeschieden wird, derart, dass eine Abgabe von im Kunststoff enthaltenen Bestandteilen an ein Produkt im Behälter verhindert wird.

Die Druckschrift WO 99/17334 A1 beschäftigt sich mit der Plasmabehandlung fertiger Behälter, wobei das zur Behandlung des Behälters genutzte Plasma während einer Evakuierung des Behälters innerhalb seines Innenraumes erzeugt und stabilisiert wird. Zur Vermeidung einer Beschädigung des Behälters wird der Innenraum gleichzeitig mit seiner unmittelbaren Umgebung evakuiert. Für die Erzeugung und Stabilisierung des Plasmas innerhalb des Behälters wird eine den Behälter umgebende Mikrowellenabschirmung mit einem Mikrowellenfeld zur Resonanz angeregt.

Aus WO 2011/138463 A1 ist ein Verfahren und eine Vorrichtung zur schnellen Dekontamination und Sterilisation von thermolabilen Gütern unter Anwendung eines Plasmas bekannt.

Bei einer Behälterformung durch Blasdruckeinwirkung werden Vorformlinge aus einem thermoplastischen Material, beispielsweise Vorformlinge aus PET (Poly-ethylenterephtalat), innerhalb einer Blasmaschine unterschiedlichen Bearbeitungsstationen zugeführt. Typischerweise weist eine derartige Blasmaschine eine Heizeinrichtung sowie eine Blaseinrichtung auf, in deren Bereich der zuvor temperierte Vorformling durch biaxiale Orientierung zu einem Behälter expandiert wird. Die Expansion erfolgt mit Hilfe von Druckluft, die in den zu expandierenden Vorformling eingeleitet wird. Der verfahrenstechnische Ablauf bei einer derartigen Expansion des Vorformlings wird in der DE-OS 43 40 291 erläutert.

Der grundsätzliche Aufbau einer Blasstation zur Behälterformung wird in der DE-OS 42 12 583 beschrieben. Möglichkeiten zur Temperierung der Vorformlinge werden in der DE - OS 23 52 926 erläutert.

Innerhalb der Vorrichtung zur Blasformung können die Vorformlinge sowie die geblasenen Behälter mit Hilfe unterschiedlicher Handhabungseinrichtungen transportiert werden. Bewährt hat sich insbesondere die Verwendung von Transportdornen, auf die die Vorformlinge aufgesteckt werden. Die Vorformlinge können aber auch mit anderen Trageinrichtungen gehandhabt werden. Die Verwendung von Greifzangen zur Handhabung von Vorformlingen und die Verwendung von Spreizdornen, die zur Halterung in einen Mündungsbereich des Vorformlings einführbar sind, gehören ebenfalls zu den verfügbaren Konstruktionen.

Eine Handhabung von Behältern unter Verwendung von Übergaberädern wird beispielsweise in der DE-OS 199 06 438 bei einer Anordnung des Übergaberades zwischen einem Blasrad und einer Ausgabestrecke beschrieben.

Die bereits erläuterte Handhabung der Vorformlinge erfolgt zum einen bei den sogenannten Zweistufenverfahren, bei denen die Vorformlinge zunächst in einem Spritzgußverfahren hergestellt, anschließend zwischengelagert und erst später hinsichtlich ihrer Temperatur konditioniert und zu einem Behälter aufgeblasen werden. Zum anderen erfolgt eine Anwendung bei den sogenannten Einstufenverfahren, bei denen die Vorformlinge unmittelbar nach ihrer spritzgußtechnischen Herstellung und einer ausreichenden Verfestigung geeignet temperiert und anschließend aufgeblasen werden.

Im Hinblick auf die verwendeten Blasstationen sind unterschiedliche Ausführungsformen bekannt. Bei Blasstationen, die auf rotierenden Transporträdern angeordnet sind, ist eine buchartige Aufklappbarkeit der Formträger häufig anzutreffen. Es ist aber auch möglich, relativ zueinander verschiebliche oder andersartig geführte Formträger einzusetzen. Bei ortsfesten Blasstationen, die insbesondere dafür geeignet sind, mehrere Kavitäten zur Behälterformung aufzunehmen, werden typischerweise parallel zueinander angeordnete Platten als Formträger verwendet.

Aufgabe der vorliegenden Erfindung ist es, ein Verfahren der einleitend genannten Art derart zu verbessern, dass in einfacher weise eine zuverlässige Sterilisation durchgeführt werden kann.

Diese Aufgabe wird erfindungsgemäß dadurch gelöst, dass die Sterilisierung nach einem Einsetzen des Vorformlings in eine Blasstation durch Anwendung eines Plasmas durchgeführt wird, wobei das Plasma außerhalb des Vorformlings erzeugt und in den Vorformling eingeleitet wird.

Weitere Aufgabe der vorliegenden Erfindung ist es, eine Vorrichtung der einleitend genannten Art derart zu konstruieren, dass eine effektive Sterilisation mit geringem Aufwand durchführbar ist.

Diese Aufgabe wird erfindungsgemäß dadurch gelöst, dass die Blasstation mit mindestens einem Plasmagenerator verbunden ist, der das Plasma mit einem räumlichen Abstand zum Vorformling erzeugt, wobei der Plasmagenerator über mindestens einen Strömungsweg mit einem Innenraum der Blasstation gekoppelt ist.

Die Sterilisierung der Vorformlinge innerhalb der Blasstation durch Anwendung eines Plasmas optimiert die Lösung aller Anforderungen, die hinsichtlich einer zuverlässigen und effektiven Sterilisierung bei der Herstellung von blasgeformten Behältern gestellt werden.

Alternativ ist auch daran gedacht, den Plasmagenerator im Bereich eines Übergabe- oder Transportrades anzuordnen und hier den Vorformling von innen und/oder von außen zu sterilisieren. Dies erfolgt alternativ oder ergänzend zur Anordnung des Plasmagenerators im Bereich des Blasrades.

Gegenüber einer Sterilisierung von geblasenen Behältern liegt eine wesentlich geringere zu sterilisierende Oberfläche bei den Vorformlingen vor. Gegenüber einer Sterilisierung von Vorformlingen im Bereich einer Heizung oder entlang eines Transportweges der Vorformlinge entfallen zusätzliche Baueinheiten. Die Verwendung des Plasmas zur Sterilisierung vermeidet das Auftreten von Resten eines Sterilisationsmittels in den geblasenen Behältern.

Durch die Sterilisation der Vorformlinge innerhalb der Blasstation wird gegenüber einer Sterilisierung der Vorformlinge im Bereich einer Heizung die Gefahr einer Rekontamination vermieden und gleichzeitig der vorteil einer geringen zu sterilisierenden Oberfläche erhalten. Innerhalb des Verfahrensablaufes der Blasformung von Behältern aus den Vorformlingen erfolgt somit zeitlich die Sterilisierung des Vorformlings im zeitlich letzten noch möglichen Moment vor der Oberflächenvergrößerung durch den Blasvorgang.

Das erfindungsgemäße Verfahren sowie die erfindungsgemäße Konstruktion ermöglichen einen äußerst kompakten Aufbau, eine preiswerte Durchführung der Sterilisation bei gleichzeitig sehr hoher Sterilisiersicherheit und unter weitgehender Vermeidung zusätzlicher Prozeßzeiten.

Gemäß einer typischen Ausführungsform ist daran gedacht, dass eine innere Oberfläche der Vorformlinge sterilisiert wird.

Eine preiswerte Erzeugung des Plasmas wird dadurch unterstützt, dass die Plasmabehandlung bei Umgebungsdruck durchgeführt wird.

Zur Bereitstellung einer kompakten Konstruktion trägt es insbesondere bei, dass das Plasma durch eine Reckstange hindurch in den Vorformling eingeleitet wird.

Zur Vermeidung oder Verringerung zusätzlicher Prozeßzeiten für die Durchführung der Sterilisierung ist insbesondere daran gedacht, dass das Plasma während eines Einfahrens der Reckstange in den Vorformling hinein in Richtung auf eine Innenwand des Vorformlings aus der Reckstange herausströmt.

Eine effektive Durchführung des Sterilisiervorganges wird dadurch unterstützt, dass das Plasma im Bereich eines in den Vorformling hineinragenden Endes der Reckstange aus der Reckstange ausströmt.

Für eine in einer Umfangsrichtung vollständige Sterilisierung ist vorgesehen, dass das Plasma aus einer Ausströmdüse der Reckstange austritt, wobei die Ausströmdüse mindestens bereichsweise schlitzförmig gestaltet wird.

Für Hochleistungsmaschinen erweist es sich als zweckmäßig, dass ein das Plasma erzeugender Plasmagenerator gemeinsam mit der Blasstation in einer umlaufenden Bewegung von einem Blasrad transportiert wird.

In den Zeichnungen sind Ausführungsbeispiele der Erfindung schematisch dargestellt. Es zeigen:
- Fig. 1: Eine perspektivische Darstellung einer Blasstation zur Herstellung von Behältern aus Vorformlingen,
- Fig. 2: einen Längsschnitt durch eine Blasform, in der ein Vorformling gereckt und expandiert wird,
- Fig. 3: eine Skizze zur Veranschaulichung eines grundsätzlichen Aufbaus einer Vorrichtung zur Blasformung von Behältern,
- Fig. 4: eine modifizierte Heizstrecke mit vergrößerter Heizkapazität,
- Fig. 5: einen schematischen Längsschnitt durch einen Vorformling, in den eine hohle Reckstange zur Zuführung eines Umgebungsdruckplasmas eingeführt ist,
- Fig. 6: einen Horizontalschnitt durch die Reckstange gemäß Schnittlinie VI in Fig. 5 und
- Fig. 7: einen Horizontalschnitt durch die Reckstange gemäß Schnittlinie VII in Fig. 5.

Vor einer Erläuterung des Detailaufbaus der Vorrichtung zum Sterilisieren der Vorformlinge (1) durch Anwendung eines Plasmas sowie vor einer Erläuterung eines konkreten Einbaues einer entsprechenden Vorrichtung in eine Blasmaschine soll nachfolgend zunächst der grundsätzliche Aufbau einer Blasmaschine beschrieben werden.

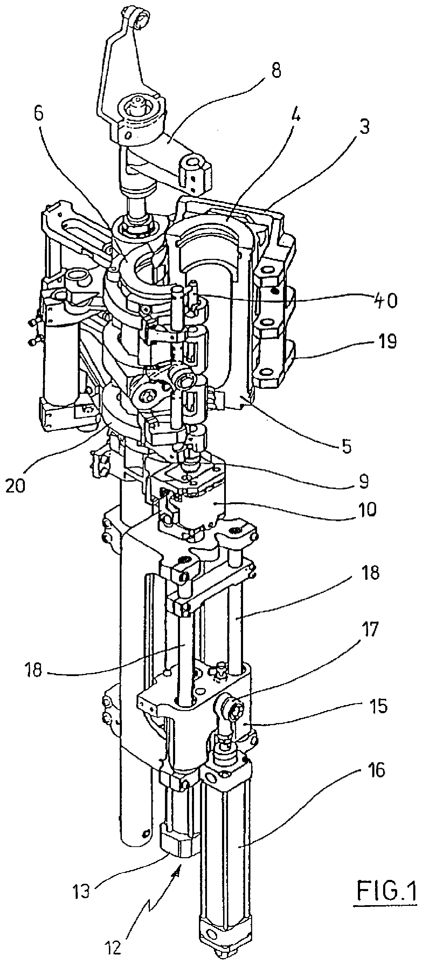

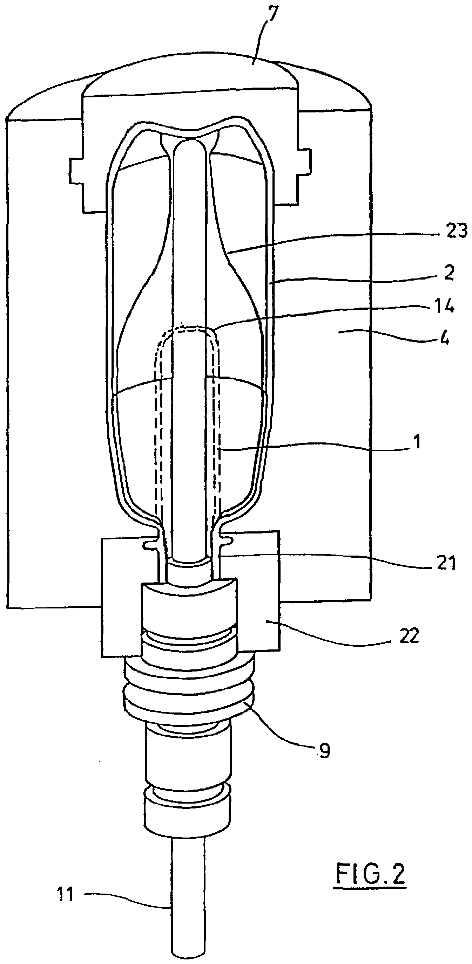

Der prinzipielle Aufbau einer Vorrichtung zur Umformung von Vorformlingen (1) in Behälter (2) ist in Fig. 1 und in Fig. 2 dargestellt.

Die Vorrichtung zur Formung des Behälters (2) besteht im wesentlichen aus einer Blasstation (3), die mit einer Blasform (4) versehen ist, in die ein Vorformling (1) einsetzbar ist. Der Vorformling (1) kann ein spritzgegossenes Teil aus Polyethylenterephthalat sein. Zur Ermöglichung eines Einsetzens des Vorformlings (1) in die Blasform (4) und zur Ermöglichung eines Herausnehmens des fertigen Behälters (2) besteht die Blasform (4) aus Formhälften (5, 6) und einem Bodenteil (7), das von einer Hubvorrichtung (8) positionierbar ist. Der Vorformling (1) kann im Bereich der Blasstation (3) von einem Transportdorn (9) gehalten sein, der gemeinsam mit dem Vorformling (1) eine Mehrzahl von Behandlungsstationen innerhalb der Vorrichtung durchläuft. Es ist aber auch möglich, den Vorformling (1) beispielsweise über Zangen oder andere Handhabungsmittel direkt in die Blasform (4) einzusetzen.

Zur Ermöglichung einer Druckluftzuleitung ist unterhalb des Transportdornes (9) ein Anschlußkolben (10) angeordnet, der dem Vorformling (1) Druckluft zuführt und gleichzeitig eine Abdichtung relativ zum Transportdorn (9) vornimmt. Bei einer abgewandelten Konstruktion ist es grundsätzlich aber auch denkbar, feste Druckluftzuleitungen zu verwenden.

Eine Reckung des Vorformlings (1) erfolgt mit Hilfe einer Reckstange (11), die von einem Zylinder (12) positioniert wird. Grundsätzlich ist es aber auch denkbar, eine mechanische Positionierung der Reckstange (11) über Kurvensegmente durchzuführen, die von Abgriffrollen beaufschlagt sind. Die Verwendung von Kurvensegmenten ist insbesondere dann zweckmäßig, wenn eine Mehrzahl von Blasstationen (3) auf einem rotierenden Blasrad angeordnet sind. Eine Verwendung von Zylindern (12) ist zweckmäßig, wenn ortsfest angeordnete Blasstationen (3) vorgesehen sind.

Bei der in Fig. 1 dargestellten Ausführungsform ist das Recksystem derart ausgebildet, dass eine Tandem-Anordnung von zwei Zylindern (12) bereitgestellt ist. Von einem Primärzylinder (13) wird die Reckstange (11) zunächst vor Beginn des eigentlichen Reckvorganges bis in den Bereich eines Bodens (14) des Vorformlings (1) gefahren. Während des eigentlichen Reckvorganges wird der Primärzylinder (13) mit ausgefahrener Reckstange gemeinsam mit einem den Primärzylinder (13) tragenden Schlitten (15) von einem Sekundärzylinder (16) oder über eine Kurvensteuerung positioniert. Insbesondere ist daran gedacht, den Sekundärzylinder (16) derart kurvengesteuert einzusetzen, dass von einer Führungsrolle (17), die während der Durchführung des Reckvorganges an einer Kurvenbahn entlang gleitet, eine aktuelle Reckposition vorgegeben wird. Die Führungsrolle (17) wird vom Sekundärzylinder (16) gegen die Führungsbahn gedrückt. Der Schlitten (15) gleitet entlang von zwei Führungselementen (18).

Nach einem Schließen der im Bereich von Trägern (19, 20) angeordneten Formhälften (5, 6) erfolgt eine Verriegelung der Träger (19, 20) relativ zueinander mit Hilfe einer Verriegelungseinrichtung (40).

Zur Anpassung an unterschiedliche Formen eines Mündungsabschnittes (21) des Vorformlings (1) ist gemäß Fig. 2 die Verwendung separater Gewindeeinsätze (22) im Bereich der Blasform (4) vorgesehen.

Fig. 2 zeigt zusätzlich zum geblasenen Behälter (2) auch gestrichelt eingezeichnet den Vorformling (1) und schematisch eine sich entwickelnde Behälterblase (23).

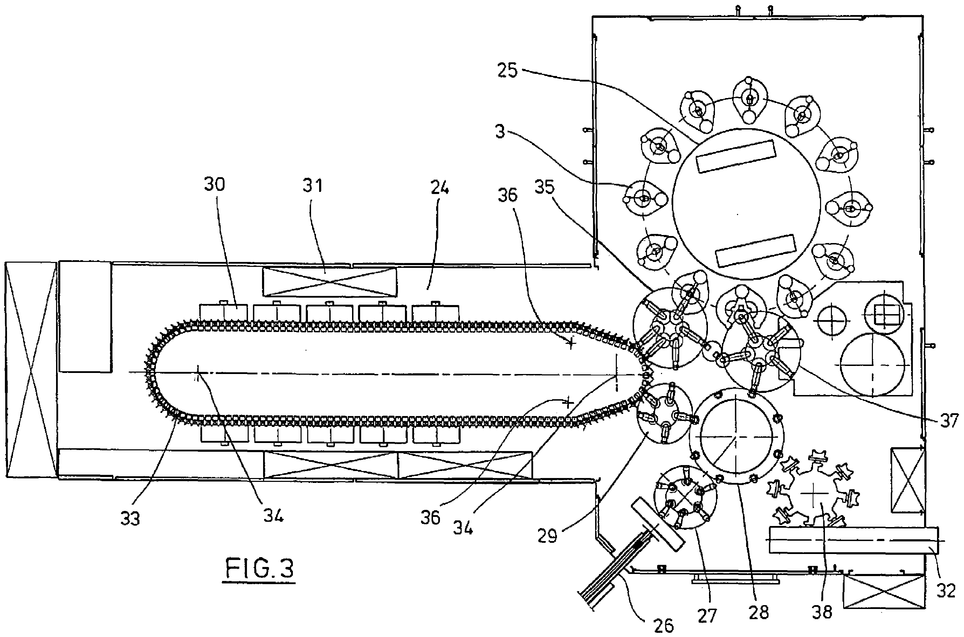

Fig. 3 zeigt den grundsätzlichen Aufbau einer Blasmaschine, die mit einer Heizstrecke (24) sowie einem rotierenden Blasrad (25) versehen ist. Ausgehend von einer Vorformlingseingabe (26) werden die Vorformlinge (1) von Übergaberädern (27, 28, 29) in den Bereich der Heizstrecke (24) transportiert. Entlang der Heizstrecke (24) sind Heizstrahler (30) sowie Gebläse (31) angeordnet, um die Vorformlinge (1) zu temperieren. Nach einer ausreichenden Temperierung der Vorformlinge (1) werden diese an das Blasrad (25) übergeben, in dessen Bereich die Blasstationen (3) angeordnet sind. Die fertig geblasenen Behälter (2) werden von weiteren Übergaberädern einer Ausgabestrecke (32) zugeführt.

Um einen Vorformling (1) derart in einen Behälter (2) umformen zu können, dass der Behälter (2) Materialeigenschaften aufweist, die eine lange Verwendungsfähigkeit von innerhalb des Behälters (2) abgefüllten Lebensmitteln, insbesondere von Getränken, gewährleisten, müssen spezielle Verfahrensschritte bei der Beheizung und Orientierung der Vorformlinge (1) eingehalten werden. Darüber hinaus können vorteilhafte Wirkungen durch Einhaltung spezieller Dimensionierungsvorschriften erzielt werden.

Als thermoplastisches Material können unterschiedliche Kunststoffe verwendet werden. Einsatzfähig sind beispielsweise PET, PEN oder PP.

Die Expansion des Vorformlings (1) während des Orientierungsvorganges erfolgt durch Druckluftzuführung. Die Druckluftzuführung ist in eine Vorblasphase, in der Gas, zum Beispiel Preßluft, mit einem niedrigen Druckniveau zugeführt wird und in eine sich anschließende Hauptblasphase unterteilt, in der Gas mit einem höheren Druckniveau zugeführt wird. Während der Vorblasphase wird typischerweise Druckluft mit einem Druck im Intervall von 10 bar bis 25 bar verwendet und während der Hauptblasphase wird Druckluft mit einem Druck im Intervall von 25 bar bis 40 bar zugeführt.

Aus Fig. 3 ist ebenfalls erkennbar, dass bei der dargestellten Ausführungsform die Heizstrecke (24) aus einer Vielzahl umlaufender Transportelemente (33) ausgebildet ist, die kettenartig aneinandergereiht und entlang von Umlenkrädern (34) geführt sind. Insbesondere ist daran gedacht, durch die kettenartige Anordnung eine im Wesentlichen rechteckförmige Grundkontur aufzuspannen. Bei der dargestellten Ausführungsform werden im Bereich der dem Übergaberad (29) und einem Eingaberad (35) zugewandten Ausdehnung der Heizstrecke (24) ein einzelnes relativ groß dimensioniertes Umlenkrad (34) und im Bereich von benachbarten Umlenkungen zwei vergleichsweise kleiner dimensionierte Umlenkräder (36) verwendet. Grundsätzlich sind aber auch beliebige andere Führungen denkbar.

Zur Ermöglichung einer möglichst dichten Anordnung des Übergaberades (29) und des Eingaberades (35) relativ zueinander erweist sich die dargestellte Anordnung als besonders zweckmäßig, da im Bereich der entsprechenden Ausdehnung der Heizstrecke (24) drei Umlenkräder (34, 36) positioniert sind, und zwar jeweils die kleineren Umlenkräder (36) im Bereich der Überleitung zu den linearen Verläufen der Heizstrecke (24) und das größere Umlenkrad (34) im unmittelbaren Übergabebereich zum Übergaberad (29) und zum Eingaberad (35). Alternativ zur Verwendung von kettenartigen Transportelementen (33) ist es beispielsweise auch möglich, ein rotierendes Heizrad zu verwenden.

Nach einem fertigen Blasen der Behälter (2) werden diese von einem Entnahmerad (37) aus dem Bereich der Blasstationen (3) herausgeführt und über das Übergaberad (28) und ein Ausgaberad (38) zur Ausgabestrecke (32) transportiert.

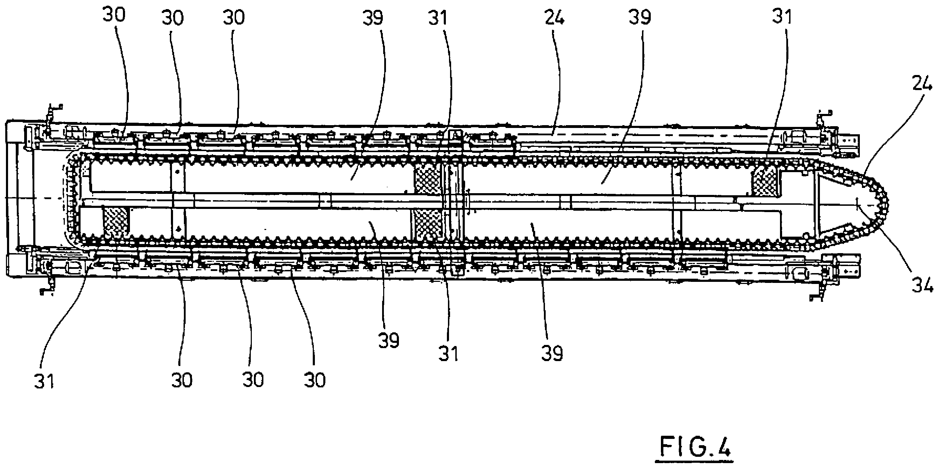

In der in Fig. 4 dargestellten modifizierten Heizstrecke (24) können durch die größere Anzahl von Heizstrahlern (30) eine größere Menge von Vorformlingen (1) je Zeiteinheit temperiert werden. Die Gebläse (31) leiten hier Kühlluft in den Bereich von Kühlluftkanälen (39) ein, die den zugeordneten Heizstrahlern (30) jeweils gegenüberliegen und über Ausströmöffnungen die Kühlluft abgeben. Durch die Anordnung der Ausströmrichtungen wird eine Strömungsrichtung für die Kühlluft im Wesentlichen quer zu einer Transportrichtung der Vorformlinge (1) realisiert. Die Kühlluftkanäle (39) können im Bereich von den Heizstrahlern (30) gegenüberliegenden Oberflächen Reflektoren für die Heizstrahlung bereitstellen, ebenfalls ist es möglich, über die abgegebene Kühlluft auch eine Kühlung der Heizstrahler (30) zu realisieren.

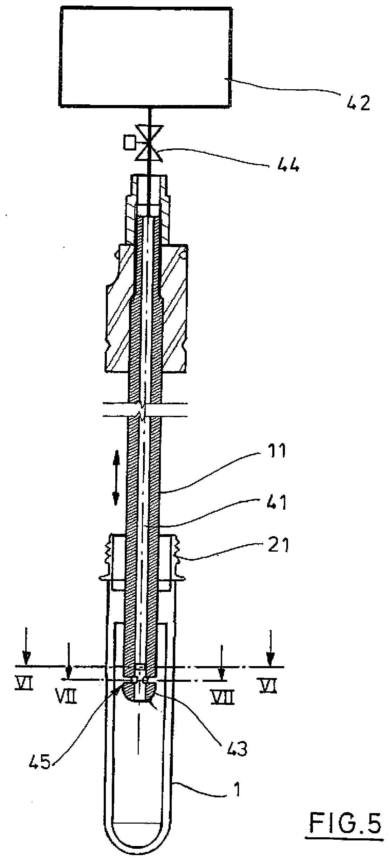

Fig. 5 zeigt einen Längsschnitt durch einen Vorformling (1), in den eine Reckstange (11) eingeführt ist, die zumindest abschnittweise hohl ausgebildet ist und einen Innenraum (41) aufweist. Der Innenraum (41) ist mit einem Plasmagenerator (42) verbunden, der typischerweise als ein Plasmajet ausgebildet ist. Der Plasmagenerator (42) generiert durch elektrische Entladungsvorgänge bei einem Druck, der zumindest näherungsweise einem Umgebungsdruck entspricht und somit etwa 1 bar beträgt, ein gasförmiges Plasma. Typischerweise wird unter Verwendung der elektrischen Entladung Luft ionisiert, die der Umgebung entnommen wird. Grundsätzlich ist es aber auch denkbar, spezielle Prozeßgase zuzuführen oder derartige Prozeßgase mit Umgebungsluft zu mischen.

Der Plasmagenerator (42) ist beim Ausführungsbeispiel gemäß Fig. 5 mit einem Ende der Reckstange (11) verbunden, das einer in den Vorformling (1) einführbaren Kuppe (43) abgewandt angeordnet ist. Ein Verbindungsweg zwischen dem Innenraum (41) der Reckstange (11) und dem Plasmagenerator (42) kann von einem Ventil (44) absperrbar sein. Ein geschlossenes Ventil (44) verhindert insbesondere eine Strömung von Blasgas aus dem Vorformling (1) bzw. dem Behälter (2) heraus in Richtung auf den Plasmagenerator (42).

Die Reckstange (11) weist eine oder mehrere Ausströmdüsen (45) auf, aus denen das Plasma in Richtung auf eine Innenwand des Vorformlings (1) austritt. Durch eine Beaufschlagung der Innenwand des Vorformlings (1) mit dem Plasma erfolgt eine Sterilisierung bzw. zumindest eine erhebliche Keimreduktion. Der Sterilisiervorgang wird typischerweise bei einem Einfahren der Reckstange (11) in den Innenraum (41) hinein durchgeführt. Hierdurch wird zusätzliche Prozeßzeit minimiert bzw. sogar vermieden.

Ein Vorschub der Reckstange (11) erfolgt typischerweise derart, dass eine Einwirkungszeit des Plasmas auf jeden Bereich der inneren Oberfläche des Vorformlings (1) für einen Zeitraum von etwa 5 Millisekunden. gewährleistet ist. In Abhängigkeit von einer Größe der Ausströmdüse (45) ergibt sich hierdurch eine Sterilisierzeit von insgesamt 50-150 Millisekunden. Eine Einfahrgeschwindigkeit der Reckstange (11) in den Vorformling (1) hinein bei der Durchführung des Sterilisiervorganges beträgt etwa 0,1 bis 2,0 m/sec. Bevorzugt ist an eine Geschwindigkeit im Bereich von 1,0 bis 2,0 m/sec. gedacht.

Ein Abstand zwischen der Ausströmdüse (45) und der Innenwand des Vorformlings (1) bei der Durchführung des Sterilisiervorganges beträgt etwa 1,0 bis 20 mm. Der Abstand wird derart gewählt, dass in Abhängigkeit von einer Strömungsgeschwindigkeit des Plasmas die sich ausbildende Plasmaflamme die innere Oberfläche des Vorformlings (1) erreichen kann.

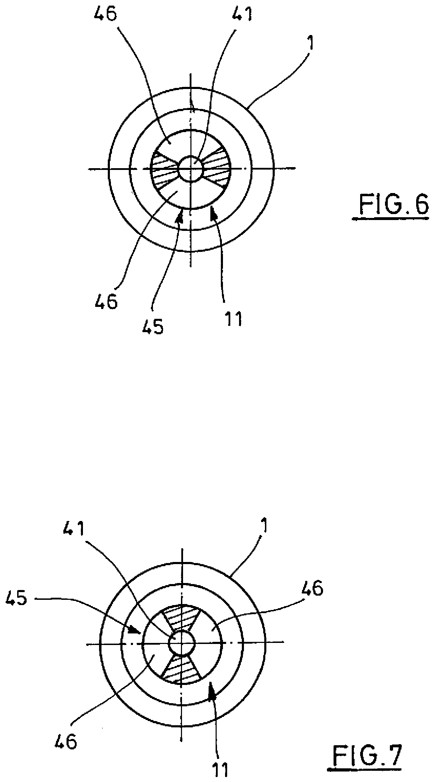

Fig. 6 und Fig. 7 zeigen Horizontalschnitte durch die Reckstange (11) im Bereich der Ausströmdüse (45). Es ist zu erkennen, dass die Ausströmdüse (45) aus einem oder mehreren Schlitzen (46) besteht. Insbesondere ist daran gedacht, in einer Längsrichtung der Reckstange (11) mindestens zwei Reihen von Schlitzen (46) übereinander anzuordnen. Fig. 6 und Fig. 7 zeigen hierbei eine Ausführungsform, bei der jede dieser Reihen aus zwei Schlitzen (46) besteht, die in einer Umfangsrichtung jeweils von einem Steg voneinander getrennt sind. Diese Konstruktion sorgt zum einen für eine erforderliche mechanische Festigkeit, darüber hinaus wird dafür gesorgt, dass entlang des gesamten Umfangsbereiches der Reckstange (11) Plasma aus dem Innenraum (41) der Reckstange (11) austreten kann.

Gemäß einer typischen Ausführungsform sind sowohl die Blasstation (3) als auch der Plasmagenerator (42) auf einem rotierenden Blasrad (45) angeordnet. Da der Plasmagenerator (42) bei Umgebungsdruck arbeitet und als zu ionisierendes Gas die Umluft verwendet, benötigt der Plasmagenerator (42) zur Versorgung lediglich elektrische Energie. Da diese ohnehin dem Blasrad (45) zugeführt wird, werden keine zusätzlichen Versorgungsanschlüsse benötigt.

Gemäß einem typischen Verfahrensablauf werden die Vorformlinge (1) in die Blasstation (3) eingesetzt und vor, während oder nach einem Schließen der Formhälften (5, 6) fährt die Reckstange (11) in den Innenraum (1) des Vorformlings (1) ein. Während des Einfahrens der Reckstange (11) generiert der Plasmagenerator (42) das erforderliche Plasma und dieses tritt aus der Ausströmdüse (45) aus, um den Sterilisiervorgang durchzuführen.

Nach einem Abschluß des Sterilisiervorganges und einem zumindest bis dahin erfolgten Schließen der Blasstation (3) erfolgt die Zuführung des Blasgases und der Vorformling (1) wird in den Behälter (2) umgeformt.

Abgesehen vom Plasmagenerator (42) und der hohl ausgebildeten Reckstange (11) werden somit keinerlei weiteren Bauelemente zur Durchführung des Sterilisiervorganges benötigt. Das Konstruktionsprinzip unterstützt insbesondere eine lediglich bedarfsabhängig erfolgende Ausstattung einer standardmäßigen Blasmaschine mit der Sterilisiereinrichtung, wenn diese für spezielle Anwendungen benötigt wird.

## Patentansprüche

1. Verfahren zum Sterilisieren von Vorformlingen aus einem thermoplastischen Material, die zur Herstellung von blasgeformten Behältern vorgesehen sind, **dadurch gekennzeichnet, dass** die Sterilisierung nach einem Einsetzen des Vorformlings (1) in eine Blasstation (3) durch Anwendung eines Plasmas durchgeführt wird, wobei das Plasma außerhalb des Vorformlings (1) erzeugt und in den Vorformling eingeleitet wird, und wobei ein das Plasma erzeugender Plasmagenerator (42) gemeinsam mit der Blasstation (3) in einer umlaufenden Bewegung von einem Blasrad (25) transportiert wird.

2. Verfahren nach Anspruch 1, **dadurch gekennzeichnet, dass** eine innere Oberfläche der Vorformlinge (1) sterilisiert wird.

3. Verfahren nach einem der Ansprüche 1 oder 2, **dadurch gekennzeichnet, dass** die Plasmabehandlung bei Umgebungsdruck durchgeführt wird.

4. Verfahren nach einem der Ansprüche 1 bis 3, **dadurch gekennzeichnet, dass** das Plasma durch eine Reckstange (11) hindurch in den Vorformling (1) eingeleitet wird.

5. Verfahren nach Anspruch 4, **dadurch gekennzeichnet, dass** das Plasma während eines Einfahrens der Reckstange (11) in den Vorformling (1) hinein in Richtung auf eine Innenwand des Vorformlings (1) aus der Reckstange (11) herausströmt.

6. Verfahren nach einem der Ansprüche 1 bis 5, **dadurch gekennzeichnet, dass** das Plasma im Bereich eines in den Vorformling (1) hineinragenden Endes der Reckstange (11) aus der Reckstange (11) ausströmt.

7. Verfahren nach einem der Ansprüche 1 bis 6, **dadurch gekennzeichnet, dass** das Plasma aus einer Ausströmdüse (45) der Reckstange (11) austritt, wobei die Ausströmdüse (45) mindestens bereichsweise schlitzförmig gestaltet wird.

8. Vorrichtung zur Blasformung von Behältern, die mindestens eine auf einer Tragstruktur angeordnete Blasstation zur Umformung von thermoplastischen Vorformlingen in die Behälter aufweist, **dadurch gekennzeichnet, dass** mindestens eine Komponente eines rotierenden Rades der Vorrichtung mit mindestens einem Plasmagenerator (42) gekoppelt ist, wobei sowohl der Plasmagenerator (42) als auch die Blasstation (3) auf einem rotationsfähigen Blasrad (25) angeordnet sind, und wobei die Blasstation (3) mit dem Plasmagenerator (42) verbunden ist, der das Plasma mit einem räumlichen Abstand zum Vorformling (1) erzeugt, und wobei der Plasmagenerator (42) über mindestens einen Strömungsweg mit einem Innenraum der Blasstation (3) gekoppelt ist.

9. Vorrichtung nach Anspruch 8, **dadurch gekennzeichnet, dass** der Plasmagenerator (42) über den Strömungsweg mit einem Innenraum (41) einer Reckstange (11) gekoppelt ist.

10. Vorrichtung nach Anspruch 8 oder 9, **dadurch gekennzeichnet, dass** der Plasmagenerator (42) als ein Plasmajet ausgebildet ist.

11. Vorrichtung nach einem der Ansprüche 8 bis 10, **dadurch gekennzeichnet, dass** die Reckstange (11) mindestens eine Ausströmdüse (45) für das Plasma aufweist.

12. Vorrichtung nach Anspruch 11, **dadurch gekennzeichnet, dass** die Ausströmdüse (45) mindestens bereichsweise schlitzartig gestaltet ist.

## Claims

1. A method for the sterilisation of preforms made of thermoplastic material, said preforms being provided for the manufacture of blow moulded containers,
**characterised in that**
- the sterilisation is carried out after the preform (1) has been inserted into a blowing station (3) by applying a plasma, wherein the plasma is generated outside the preform (1) and is introduced into the preform, and wherein, along with the blowing station (3), a plasma generator (42) generating the plasma is transported in a circumferential movement by a blow moulding wheel (25).

2. The method according to Claim 1, **characterised in that** an inner surface of the preforms (1) is sterilised.

3. The method according to any one of Claims 1 or 2, **characterised in that** the plasma treatment is carried out at ambient pressure.

4. The method according to any one of Claims 1 to 3, **characterised in that** the plasma is introduced into the preform (1) through a stretch forming bar (11).

5. The method according to Claim 4, **characterised in that** the plasma flows out of the stretch forming bar (11) while the stretch forming bar (11) moves into the preform (1) towards an inner wall of the preform (1).

6. The method according to any one of Claims 1 to 5, **characterised in that** the plasma flows out of the stretch forming bar (11) in the vicinity of an end of the stretch forming bar (11) that protrudes into the preform (1).

7. The method according to any one of Claims 1 to 6, **characterised in that** the plasma exits from a flow-out nozzle (45) of the stretch forming bar (11), wherein the flow-out nozzle (45) is being designed in the form of a slot at least in certain regions.

8. An apparatus for the blow moulding of containers, the apparatus comprising at least one blowing station that is arranged on a support structure and is intended to mould thermoplastic preforms to obtain the containers, **characterised in that** at least one component of a rotating wheel of the apparatus is coupled to at least one plasma generator (42), wherein both the plasma generator (42) and the blowing station (3) are arranged on a rotatable blow moulding wheel (25), and wherein the blowing station (3) is connected to the plasma generator (42) which generates the plasma at a spatial distance from the preform (1), and wherein the plasma generator (42) is coupled to an interior region of the blowing station (3) via at least one flow path.

9. The apparatus according to Claim 8, **characterised in that** the plasma generator (42) is coupled to an interior region (41) of a stretch forming bar (11) via the flow path.

10. The apparatus according to Claim 8 or 9, **characterised in that** the plasma generator (42) is formed as a plasma jet.

11. The apparatus according to any one of Claims 8 to 10, **characterised in that** the stretch forming bar (11) has at least one flow-out nozzle (45) for the plasma.

12. The apparatus according to Claim 11, **characterised in that** the flow-out nozzle (45) is designed in the form of a slot at least in certain regions.

## Revendications

1. Procédé de stérilisation de préformes en un matériau thermoplastique destinées à la fabrication de récipients moulés par soufflage, **caractérisé en ce que** la stérilisation a lieu, après une introduction de la préforme (1) dans une station de soufflage (3), par l'utilisation d'un plasma, le plasma étant produit à l'extérieur de la préforme (1) puis introduit dans la préforme, et un générateur de plasma (42) produisant le plasma étant transporté conjointement avec la station de soufflage (3) en un mouvement périphérique par une roue de soufflage (25).

2. Procédé selon la revendication 1, **caractérisé en ce qu'**une surface intérieure des préformes (1) est stérilisée.

3. Procédé selon l'une des revendications 1 ou 2, **caractérisé en ce que** le traitement au plasma est appliqué à pression ambiante.

4. Procédé selon l'une des revendications 1 à 3, **caractérisé en ce que** le plasma est introduit dans la préforme (1) à travers une barre d'étirage (11).

5. Procédé selon la revendication 4, **caractérisé en ce que** le plasma afflue pendant l'introduction de la barre d'étirage (11) dans la préforme (1) et est éjecté hors de la barre d'étirage (11) en direction d'une paroi intérieure de la préforme (1).

6. Procédé selon l'une des revendications 1 à 5, **caractérisé en ce que** le plasma est éjecté de la barre d'étirage (11) au niveau d'une extrémité de la barre d'étirage (11) pénétrant dans la préforme (1).

7. Procédé selon l'une des revendications 1 à 6, **caractérisé en ce que** le plasma sort d'une buse d'éjection (45) de la barre d'étirage (11), la buse d'éjection (45) étant, sur une partie au moins, réalisée sous forme de fente.

8. Dispositif de moulage par soufflage de récipients, ce dispositif présentant au moins une station de soufflage agencée sur une structure de support pour la transformation des préformes thermoplastiques en récipients, **caractérisé en ce qu'**un composant au moins d'une roue en rotation du dispositif est couplé à au moins un générateur de plasma (42), le générateur de plasma (42) aussi bien que la station de soufflage (3) étant agencés sur une roue de soufflage (25) pouvant tourner et la station de soufflage (3) étant reliée au générateur de plasma (42) qui produit le plasma en une position située à une certaine distance de la préforme (1), et le générateur de plasma (42) étant relié à un espace intérieur de la station de soufflage (3) par l'intermédiaire d'au moins une voie d'écoulement.

9. Dispositif selon la revendication 8, **caractérisé en ce que** le générateur de plasma (42) est relié à un espace intérieur (41) d'une barre d'étirage (11) par l'intermédiaire de la voie d'écoulement.

10. Dispositif selon la revendication 8 ou 9, **caractérisé en ce que** le générateur de plasma (42) est réalisé sous forme de plasmajet.

11. Dispositif selon l'une des revendications 8 à 10, **caractérisé en ce que** la barre d'étirage (11) présente au moins une buse d'éjection (45) pour le plasma.

12. Dispositif selon la revendication 11, **caractérisé en ce que** la buse d'éjection (45) est, sur une partie au moins, réalisée sous forme de fente.
